# EUROPEAN PATENT APPLICATION

(11) **EP 3 742 203 A1**
(43) Date of publication of application: **25.11.2020**
(21) Application number: 19175572.7
(22) Date of filing: 21.05.2019
(51) Int. Cl.: G01T 1/161, G01T 1/164

(54) **X-RAY AND GAMMA IMAGING USING A SINGLE RADIATION DETECTOR**

(71) Applicant: Koninklijke Philips N.V., 5656 AG Eindhoven (NL)
(72) Inventor: WIECZOREK, Herfried Karl, 5656 AE Eindhoven (NL); STEADMAN BOOKER, Roger, 5656 AE Eindhoven (NL); ALVING, Peter Lex, 5656 AE Eindhoven (NL)
(74) Representative: de Haan, Poul Erik

(57) **Abstract**

The invention relates to a system for imaging an object in an x-ray imaging mode and in a gamma imaging mode. A radiation detector (1) of the system comprises a conversion unit (202) including a plurality of detector pixels (206_{1,...,M}) and generating for each detection event a detection signal indicative of an energy of the event, and a counting unit (203) including for each detector pixel (206_{1,...,M}) a plurality of comparators (209_{i;1,...,N}) and associating each detection event to one of a plurality of predetermined energy bins based on the detection signals using the comparators (209_{i;1,...,N}). In the x-ray imaging mode, the comparators (209_{i;1,...,N}) of one pixel (206_{1,...,M}), and, in the gamma imaging mode, the comparators (209_{i;1,...,N}) of several pixels (206_{1,...,M}) are available for the association so that more energy bins are available in the gamma imaging mode than in the x-ray imaging mode.

## Description

### FIELD OF THE INVENTION

The invention relates to the acquisition of x-ray and gamma images of objects. More specifically, the invention relates to an imaging system which is operable in an x-ray imaging mode to detect x-ray photons having traversed an object and which is operable in a gamma imaging mode to detect gamma photons emitted by the object. Further, the invention relates to an imaging method for imaging an object using such an imaging system.

### BACKGROUND OF THE INVENTION

In various medical imaging procedures, it is beneficial to provide both an x-ray image and a nuclear image of a region of interest. The x-ray image typically provides structural information indicative of the anatomy of the region of interest. The nuclear image, defined herein to mean an image indicative of radiotracer distribution in an object, is generated based on detected gamma quanta. The nuclear image may, for example, be a gamma scintigraphy or a SPECT image and typically provides functional or physiological information relating to the region of interest. Together, the two different image types can be used to improve the identification of an underlying pathology during a medical investigation.

In order to acquire three-dimensional x-ray and nuclear images in one imaging examination, combined x-ray and gamma imaging system have been developed, in which an x-ray source (for x-ray imaging) and a detector assembly are mounted on a gantry, such as a C-arm, which can be rotated around the patient. Conventionally, the detector assembly includes separate radiation detectors for the detection of x-ray radiation and gamma radiation. Preferably, these detectors are configured as so-called spectral detectors or energy-binning detectors, which are capable of detecting incident x-ray or gamma photons individually and determining their energies in accordance with a certain number (e.g. two or three) of energy ranges, which are also referred to as energy bins. An example of a corresponding detector assembly, which includes stacked x-ray and gamma detectors, is described in WO 2017/007326.

However, due to the use of two detectors, such systems are very complex and expensive. Detector assemblies including two detectors are itself quite complex and costly. Further, detector assemblies including two detectors are heavier than conventional detector assemblies used in x-ray or gamma imaging, which include only a single detector. Therefore, combined x-ray and gamma imaging systems have to be provided with more solid gantries compared to those used in conventional x-ray and gamma imaging.

In view of this, it would be desirable to perform combined x-ray and gamma imaging using an imaging system having only a single detector. For instance, such a detector could be used to alternately detect x-ray photons and gamma photons in successive time intervals, as also suggested in the aforementioned WO 2017/007326 in connection with the combined stacked detector assembly.

Indeed, known spectral direct conversion detectors are capable of detecting both x-ray and gamma radiation. However, x-ray imaging often requires a relatively high spatial resolution. In order to achieve this, x-ray detectors typically have a pixel size of 150 µm or smaller. With such small pixel sizes, the detector may suffer from charge sharing and escape photons generated due to K-fluorescence (so called K-escape). These effects particularly result in inaccuracies in the determination of the energies of the x-ray photons, which are also referred as spectral distortions. However, these spectral distortions only affect a relatively small fraction of detection events and may efficiently be corrected for using model-based correction approaches.

Such approaches can also be applied in case of a high photon flux, which is typical for x-ray imaging, when other correction approaches, which evaluate individual detection events, are not applicable. These approaches are based on the detection of coincident events in neighboring pixels which are caused by charge sharing and cannot be applied in case a high number of photons are incident into the detector so that it is not possible to distinguish between coincidences due to the charge sharing and coincidences due to several photons which arrive at the detector simultaneously.

An additional effect resulting in escape photons is Compton scattering in the detector. A photon undergoing Compton scattering transfers part of its initial energy to the detector material in a certain pixel (namely to an electron in this material) and, upon the scattering event, it travels further with a reduced energy and is typically only detected in another pixel. The cross section of Compton scattering increases with the photon energy. For lower energy x-ray imaging with photon energies up to about 70 keV, this cross section is relatively small compared with the cross section of the photoabsorption in the detector material so that Compton scattering is usually negligible. However, this is no longer possible in gamma imaging due to the higher energies of the gamma photons.

For instance, if Tc-99m is used as radiotracer material, the gamma photons have an energy of about 150 keV, and for such a photon energy, a considerable fraction of photons undergoes Compton scattering. So, the ratio between the cross sections of Compton scattering and photoabsorption is 19% for 150keV photons in cadmium telluride (CdTe) which is a material often used in direct conversion detectors. Thus, taking also charge sharing and K escape into consideration, a large fraction of gamma photons (up to about 50%) is not detected correctly.

Since the photon flux is relatively small in gamma imaging, it would, in principle, be possible to correct for all the aforementioned effects by combining coincident detection events - which likely result from a single incident photon in case of a small photon flux - and summing up their energies. However, given that the detection events may generally have arbitrary individual energies, this would require an energy resolution which is higher than the energy resolution provided by the conventional energy-binning spectral radiation detectors.

One solution to this problem would be to use a single radiation detector in x-ray and gamma imaging, which has a sufficiently high energy resolution. However, this would require a large number of energy bins and related comparators for associating the detection events with these energy bins. The integration of these comparators would render the radiation detector more complex, bulky and costly.

### SUMMARY OF THE INVENTION

It is an object of the invention to allow for an accurate x-ray and gamma imaging using a single detector, which is configured less complex.

In accordance with a first aspect, the invention suggests an imaging system for imaging an object, which includes an x-ray source and a spectral radiation detector for detecting x-ray and gamma photons and which is operable in an x-ray imaging mode, in which the radiation detector is operated to register detection events caused by incident x-ray photons emitted by the x-ray source and having traversed the object, and in a gamma imaging mode, in which the radiation detector is operated to register detection events caused by incident gamma photons emitted by the object. The radiation detector comprises (i) a conversion unit including a plurality of detector pixels and configured for generating, for each detection event, a detection signal indicative of an energy of the detection event, and (ii) a counting unit including for each detector pixel a plurality of comparators and configured for associating each detection event to one of a plurality of predetermined energy bins on the basis of the detection signals using the comparators. In the x-ray imaging mode, the comparators of one pixel, and, in the gamma imaging mode, the comparators of several pixels are available for the association so that the number of energy bins available for the association in the gamma imaging mode is larger than the number of energy bins available for the association in the x-ray imaging mode.

Since the number of comparators available for associating the detection events with energy bins and, thus, the number of energy bins for the association to the detection events are greater in the gamma imaging mode, the radiation detector provides a high energy resolution in the gamma imaging mode. Moreover, since the comparators of several pixels are available for the association of individual detection events to energy bins in the gamma imaging mode, the radiation detector only requires a relatively small number of comparators per pixel in order to achieve the high energy resolution of the gamma imaging mode. In particular, the radiation detector only needs to have the number of comparators per pixel which are used for energy binning during x-ray imaging.

Each of the comparators may be configured to compare a value of the detection signal with a threshold value corresponding to one of the energy bins. The threshold value may particularly correspond to a lower end value of the energy bin. Thus, a detection event of an energy in a certain energy bin causes a detection signal of the comparator comparing the detection signal with the threshold value corresponding to the energy bin. In addition, the detection event will cause detection signals of the comparators comparing the detection signal with thresholds corresponding to lower energy bins. Thus, the detection event may be associated with the energy bin corresponding to the highest thresholds exceed by the detection signal.

In one embodiment of the invention, the radiation detector comprises a switch assembly for interconnecting a set of pixels to form a superpixel producing a related detection signal in response to each detection event occurring in the set of pixels and for coupling all comparators associated with the pixels included in the set to receive the detection signal in the gamma imaging mode. In this manner, the comparators of several pixels (i.e. the pixels included in a superpixel) can be made available for the energy determination of individual detection events in the gamma imaging mode. As a consequence, the spectral resolution is increased in the gamma imaging mode. The spatial resolution is decreased since the superpixels are the smallest spatial unit for localizing the detection events due to the interconnection of the pixels. However, this is acceptable in gamma imaging, since gamma imaging usually requires a smaller spatial resolution compared with x-ray imaging.

In a related embodiment of the invention, each pixel produces a detection signal in response to a detection event occurring therein and only the comparators associated with the respective pixel receive the detection signal in the x-ray imaging mode. As a consequence, the energy resolution is smaller in the x-ray imaging mode compared with the gamma imaging mode, but the spatial resolution is higher since the pixels form the smallest spatial units for localizing detection events in the x-ray imaging mode.

In one embodiment of the invention, the imaging system further comprises a clustering unit configured to detect a cluster event comprising detection events essentially simultaneously detected at neighboring locations of the radiation detector and to assign an energy value to each detected cluster event on the basis of the energy bins associated with the detection events included in the cluster event in the gamma imaging mode. In a related embodiment, the clustering unit is configured to detect a cluster event comprising detection events essentially simultaneously detected in neighboring superpixels.

The clustering of events allows for correcting for charge sharing between superpixels, K-escape and Compton scattering in the detector material in the gamma imaging mode. In this mode, the photon flux is usually relatively small so that a reliable clustering of detection events is possible. Due to the small photon flux, it can reliably be assumed that detection events occurring essentially simultaneously are caused by a single incident photon rather than multiple photons incident into the radiation detector simultaneously.

The energy value assigned to the cluster event may correspond to a sum of predetermined energy values of the energy bins associated with the detection events included in the cluster event, particularly to a sum of center values of the energy bins. Due to the increased energy resolution in the gamma imaging mode, this approach allows for an accurate estimation of the overall energy value of a cluster event, which corresponds to the energy of the incident photon.

In one embodiment of the invention, the clustering unit is configured to assign a virtual detection location to a cluster event on the basis of the locations at which the detection events included in the cluster event are detected. In a related embodiment, the virtual location assigned to a cluster event corresponds to a location at which the detection event with the highest associated energy among the detection events included in the cluster event is detected. In particular, the clustering unit may be configured to assign a virtual detection location to a cluster event on the basis of the superpixels in which the detection events included in the cluster event are detected. The virtual location assigned to a cluster event may particularly correspond to the superpixels which registered the detection event with the highest energy among the detection events included in the cluster event.

When the location or superpixel, where the detection event with the highest energy is registered, is determined as the virtual location of the cluster event, the actual location of the entrance of the photon into the radiation detector can often be determined, because the greatest part of the photon energy is often deposited in the radiation detector during the first interaction between the photon and the detector material.

In one embodiment of the invention, the spectral radiation detector comprises an application-specific integrated circuit (ASIC) including the counting unit and the clustering unit. In alternative embodiments, the clustering unit may be integrated into a further unit which is separated from the ASIC of the radiation detector.

In a further embodiment of the invention, the system is operable in a hybrid imaging mode, in which the x-ray source and the radiation detector rotate around the object to be imaged and register detection events at a plurality of angular positions, at each position the radiation detector being operated in the x-ray imaging mode in a first time interval and in the gamma imaging mode in a second time interval. In a related embodiment, the system is configured to operate the x-ray source only in the first time interval at each angular position. These embodiments allow for acquiring an x-ray image and a gamma image of an object in a single imaging examination.

In accordance with a further aspect, the invention suggests an imaging method for imaging an object using an imaging system including an x-ray source and a spectral radiation detector for detecting x-ray and gamma photons, the system being operable in an x-ray imaging mode, in which the radiation detector is operated to register detection events caused by incident x-ray photons emitted by the x-ray source and having traversed the object, and in a gamma imaging mode, in which the radiation detector is operated to register detection events caused by incident gamma photons emitted by the object. The method comprises: (i) a conversion unit of the radiation detector, which includes a plurality of detector pixels, generating for each detection event a detection signal indicative of an energy of the detection event, and (ii) a counting unit, which includes for each detector pixel a plurality of comparators, associating each detection event to one of a plurality of predetermined energy bins on the basis of the detection signals using the comparators. In the x-ray imaging mode, the comparators of one pixels, and, in the gamma imaging mode, the comparators of several pixels are used for the association so that the number of energy bins available for the association in the gamma imaging mode is larger than the number of energy bins available for the association in the x-ray imaging mode.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 schematically and exemplarily illustrates components of a combined x-ray and gamma imaging system, and
Fig. 2 schematically and exemplarily illustrates components of a radiation detector of the system.

### DETAILED DESCRIPTION OF EMBODIMENTS

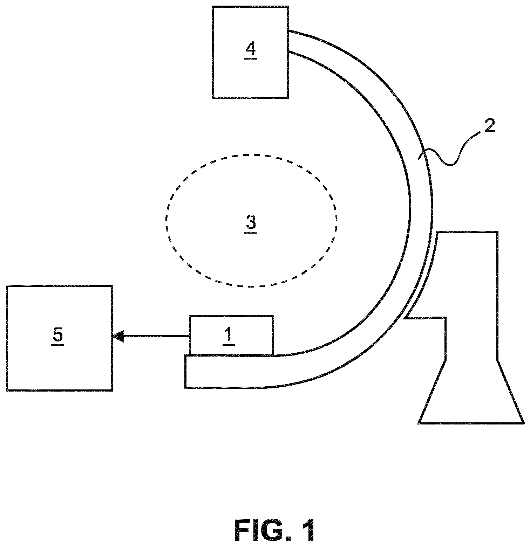

Fig. 1 schematically and exemplarily shows components of a combined x-ray and gamma imaging system, which includes a single direct conversion radiation detector 1 for detecting x-ray and gamma photons. The radiation detector 1 is configured as photon-counting spectral detector, which is capable of detecting individual photon events and assigning each detection event to one of a plurality of energy bins. The energy bins correspond to predetermined energy intervals. The system may particularly be used in medical applications in order to acquire three-dimensional x-ray and nuclear images of regions of patient bodies. However, the system may likewise be utilized in order to image other objects.

Since the radiation detector 1 is configured as a spectral detector, the system is capable of generating energy-selective x-ray images. For instance, these images allow for distinguishing between different materials of the object - this usually also referred to as material decomposition. These images may be provided for a separate evaluation, or they may be combined to generate overall images. As known to the person skilled in the art, suitable combinations of such images particularly allow to compensate for beam-hardening artifacts occurring in x-ray imaging. In the process of generating nuclear images, the energy information provided by the radiation detector can particularly be used for determining the energies of cluster events comprising multiple detection events, which are generated in response to a single photon incident into the radiation detector 1, as will be explained in more detail herein below.

The radiation detector 1 is mounted on a gantry 2, which can be rotated around an examination region 3 and which may be configured as a C-arm, as schematically illustrated in Fig. 1. In addition to the radiation detector 1, the combined imaging system comprises an x-ray source 4, which may particularly be configured as an x-ray tube, and which is likewise mounted on the gantry 2. On the gantry 2, the x-ray source 4 and the radiation detector 1 are arranged at opposition positions so that x-ray beams generated by the x-ray source 4 are collected in the radiation detector 1 upon having traversed the examination region 3 and the patient body or another object positioned therein.

The radiation detector 1 is connected to a reconstruction unit 5, which is capable of reconstructing three-dimensional images on the basis of the measurement data collected by the radiation detector in a manner known to the person skilled in the art as such. The reconstruction unit 5 may be configured as a computer device that comprises one or more processor units to execute computer programs implementing suitable image reconstruction routines.

In one mode of operation, the system is capable of acquiring three-dimensional x-ray images of regions of patient bodies or other objects positioned in the examination region 3. In this mode, the x-ray source 4 is operated to emit x-ray beams at several angular positions while the gantry 2 is rotated around the object, and the radiation detector 1 detects the radiation having traversed the object in order to obtain projection values. On the basis of these projection values, a three-dimensional image of the object in the reconstruction unit 5. Using the information about the detection locations and the energies of the photons detected by the radiation detector 1, the reconstruction unit 5 may generate energy-selective x-ray images, e.g. for each energy bin. As said above, these images may be further evaluated separately or combined to form an overall image.

In a further mode of operation, the system is capable of acquiring and three-dimensional nuclear images of the objects positioned in the examination region 3. In this mode, the x-ray source 4 is not operated to emit x-ray radiation. Rather, a gamma-emitting radioisotope is delivered into the object and the radiation detector 1 is operated to detect gamma photons emitted by the radioisotope so that the generated nuclear images show the distribution of the radioisotope in the object. In medical applications, the radioisotope may particularly be included in a material attaching to a specific type of tissue so that the spatial distribution of this tissue is shown in the nuclear images. In order to acquire three-dimensional nuclear images, the radiation detector 1 detects projection values of the object at several angular positions, while the gantry 2 is rotated around the object and the reconstruction unit 5 reconstructs the image from these values.

During the acquisition of x-ray images, the radiation flux incident into the radiation detector 1 is usually significantly higher than the radiation flux incident into the radiation detector during the acquisition of nuclear images. So, the photon count rate may be in the range of some kcps/mm² or may even be (significantly) higher during the acquisition of x-ray images, while typical count rates during the acquisition of nuclear images are only in the range of 10 cps/mm² (cps = counts per second).

This discrepancy allows for operating the system in a hybrid imaging mode, in which x-ray images and nuclear images of an object are acquired in one imaging examination. In the hybrid imaging mode, the images are acquired after the radioisotope has been delivered to the object to be imaged. Further, in order to acquire the x-ray and nuclear images, the radiation detector 1 is again operated to detect photons at several angular positions, while the gantry 2 is rotating around the object. At each angular position, an x-ray projection and a gamma projection are acquired. The x-ray projections correspond to two-dimensional x-ray images which may be used in order to reconstruct a three-dimensional x-ray image of the object. The gamma projections correspond to two-dimensional nuclear images which are used in order to generate a three-dimensional nuclear image of the object.

For acquiring an x-ray projection at a certain angular position of the gantry 2, the x-ray source 4 is operated during a first time interval, and the photons incident onto the radiation detector 1 are detected. The first time interval is selected relatively short and may have a length between 5 ms and 10 ms, for example. During a time interval of this length, a sufficiently high number of x-ray photons is detected in the radiation detector 1 in order to be able to create an accurate x-ray projection of the object, while no or only few gamma photons reach the radiation detector 1.

The gamma projection is acquired in a second time interval before or after the acquisition of the x-ray projection. During the second time interval, the x-ray source 4 does not emit x-ray radiation so that only gamma photons reach the radiation detector 1. In order to collect a sufficiently high number of gamma photons for creating the gamma projection, the second time interval may be selected significantly longer than the first time interval. In particular, the second time interval may have a length between some ten milliseconds and some ten seconds.

As will be explained in more detail herein below, the radiation detector 1 used in the system can be operated in two modes of operation, an x-ray imaging mode for acquiring x-ray images and a gamma imaging mode for acquiring nuclear images of an object. The x-ray imaging mode is activated when the radiation detector 1 is operated to detect x-ray photons emitted by the x-ray source 4 in order to acquire x-ray images, as explained above. In this mode, the radiation detector 1 determines the energy of the incident photons in accordance with a certain number of energy bins. The gamma imaging mode is activated when the radiation detector is operated to detect gamma photons emitted by a radioisotope introduced into the object to be imaged in order to acquire nuclear images of the object. In the gamma imaging mode, it is particularly possible to determine the energies of photons incident into the radiation detector 1 in accordance with a higher number of energy bins and, thus, with a higher energy resolution. This higher energy resolution is particularly used in order to determine clusters of detection events which relate to a single photon incident into the radiation detector 1.

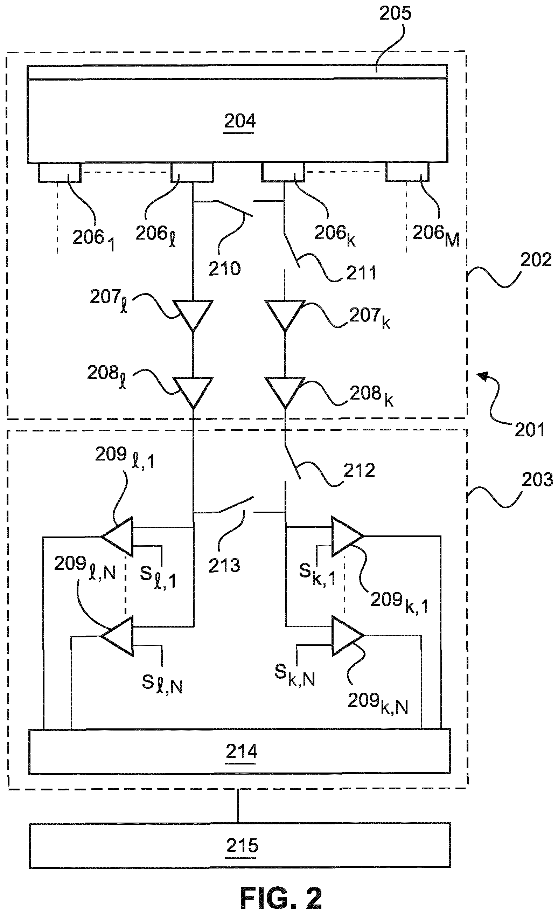

Components of an embodiment of the radiation detector 1 are schematically and exemplarily illustrated in Fig. 2. In this embodiment, the radiation detector 1 comprises a plurality of detector elements 201, which are usually also referred to as tiles and which are preferably arranged in an array that may be flat or concave. Thus, the detector elements 201 are arranged in the form of rows and columns arranged substantially perpendicular to each other. In Fig. 2, only one of these detector elements 201 is shown by way of example.

Each detector element 201 comprises a conversion unit 202, which has a plurality of pixels for detecting events and generating a detection signal (e.g. a voltage signal) for each event detected in one of the pixels, which is indicative of the energy of the detection event. Moreover, each detector element 201 includes a counting unit 203, which is configured to associate each detection event to one of a plurality of energy bins on the basis of the detecting signals and to determine the numbers of detection events associated with each energy bin in each of a plurality of successive time frames.

The conversion unit 202 comprises a converter element 204 for converting photons into electrical signals. The converter element 204 is provided between a cathode contact 205 and an anode contact assembly 206_{1,...,M} and is made of a semiconductor material. Suitable semiconductor materials include, for example, cadmium telluride (CdTe), cadmium zinc telluride (CdZnTe), cadmium tellurium selenide (CdTeSe), CdZnTeSe, cadmium manganese telluride (CdMnTe), silicon (Si), gallium arsenide (GaAs), organic-inorganic perovskites (MA-PbI3 or MA-PbBr3 and others) and mercury iodide (Hgl). In operation, the cathode contact 205 is generally held on a lower electric potential than the anode contact assembly 206_{1,...,M} (i.e. a negative bias voltage is applied to the cathode contact assembly 205 with respect to the anode contact assembly 206_{1,...,M}) so that an electric field is formed between the cathode contact 205 and the anode contact assembly 206_{1,...,M} within the converter element 204.

The cathode contact assembly 205 may be configured as a continuous cathode electrode, which may be formed by a thin metalized film applied onto the converter element 204. In contrast, the anode contact assembly 206_{1,...,M} may include M pixelated anode electrodes, i.e. separated anode electrodes, which are arranged in certain distances to each other and which are also referred to as anode pixels or pixels herein. In one embodiment, these anode pixels 206_{1,...,M} are also arranged in rows and columns, which are substantially perpendicular to each other, on the surface of the converter element 204.

Photons may enter into the converter element 204 through the cathode contact 205. When a photon with sufficiently high energy (e.g. an x-ray or gamma photon) enters into the converter element 204, it excites the semiconductor material and thereby generates a cloud of electric charge carriers (electrons and holes). The negative charge carriers drift to one of the anode pixels 206_{1,...,M} under the influence of the electric field in the converter element 204 and produce a pulse-like electric signal, which is collected by processing circuitry coupled to the converter element 204.

As shown in Fig. 2 for two anode pixels 206ₗ and 206ₖ, the processing circuitry may include for each of the anode pixels 206_{1,...,M} an amplifier 207ᵢ (i = 1,...,M) such as a charge sensitive amplifier, which integrates the input current over each pulse (i.e. each detection event) and produces a corresponding step-like output voltage signal, where the height of the step corresponds to the integrated charge and, thus, to the energy deposited in the converter element 204 in a detection event. The amplifier output signal is preferably filtered in a so-called pulse shaper circuit 208i to produce a detection voltage signal in which each step generated by the amplifier 207i corresponds to a pulse having a certain width and a gradually rounded maximum, the amplitude of which is proportional to the height of the step (i.e. to the integrated charge of the charge cloud generated by an incident x-ray photon) and, thus, to the energy of the incident photon.

The detection signals generated in the conversion unit 202 of a detector element 201 are further processed in the counting unit 203 of the detector element. The counting unit 203 is implemented in further processing circuitry of the detector element 201, which is integrated into an ASIC together with the processing circuitry of the conversion unit 202 (i.e. the amplifiers 207ᵢ and pulse shapers 208ᵢ provided for the anode pixels 206_{1,...,M}).

As illustrated in Fig. 2 for the anode pixels 206ₗ and 206ₖ by way of example, the counting unit 203 comprises one set of N comparators 209_{i;1,...,N} for each pixel 206_{1,...,M}. In the x-ray imaging mode, these comparators 209_{i;1,...,N} receive the detection signals of the associated pixel 206_{1,...,M}. For this purpose, the comparators 209_{i,1,...,N} are coupled to the output of the pulse shaper 208ᵢ associated with the respective anode pixel 206ᵢ. Each of the comparators 209_{i;1,...,N} compares the detection signals of the respective pixel 206ᵢ with an associated predetermined threshold value S_{i;1,...,N} and produces an output signal pulse, (only) if the detection signal exceeds the threshold value S_{i;1,...,N}.

The threshold values S_{i;1,...,N} of the comparators 209_{i;1,...,N} represent the boundaries of the aforementioned energy bins. They may be selected such that a pulse output by the shaper circuit 208ᵢ, which corresponds to a detection event having an energy within a range corresponding to a certain energy bin, has an amplitude between the threshold values corresponding to the upper and lower boundaries of the energy bins. Thus, each of the comparators 209_{i;1,...,N} is effectively associated with the energy bin having a lower boundary corresponding to the threshold value S_{i;1,...,N} associated with the respective comparator 209 _{i;1,...,N}. In response to a detection event having an energy within a certain energy bin, the associated comparator 209_{i;1,...,N} and the comparators 209_{i;1,...,N} associated with lower energy bins produce an output pulse. Therefore, the energy bin to be allocated to each detection event corresponds to the energy bin associated to the comparator 209_{i;1,...,N} with the highest threshold that produces an output pulse.

In the x-ray imaging mode, the same energy thresholds S_{i;1,...,N} are provided for the comparators 209_{i;1,...,N} of the different pixels 206_{1,...,M}. The actual threshold values may differ, e.g. due to different amplifier gains in the pixels, but the thresholds preferably correspond to the same energy values. For each anode pixel 206_{1,...,M}, the counting unit 203 may include a relative small number N of comparators 209_{i;1,...,N}, where N may be between 2 and 6, for example. As a consequence, the number of available energy bins, is likewise relative small.

Such a small number of energy bins usually allows for a sufficient material decomposition of the object in medical applications, for example. In this case, it is sufficient that the number of energy bins corresponds to the number of materials that shall be distinguished and the end values of the energy bins may be set in accordance with the spectral attenuation characteristics of the materials to be distinguished and the photon energies used in the imaging process for this purpose. However, the number of energy bins corresponding to the comparators 209_{i;1,...,N} provided per pixel 206ᵢ is usually too small for the clustering of detection events, which is particularly carried out in gamma imaging, as will be explained in more detail herein below.

In order to increase the number of available energy bins in gamma imaging, the radiation detector 1 is configured such that multiple pixels 206i can be interconnected in such a way that all comparators 209_{i;1,...,N} associated with the interconnected pixels can be used for determining the energies of events detected in the interconnected pixels. Thus, when the interconnected pixels - which are also referred to as superpixel herein - include P pixels, P x N comparators 209_{i;1,...,N} and, thus, P x N energy thresholds are available for determining the energies of events. In practical implementations, a superpixel may consist of 4 to 64 individual pixels 206ᵢ so that 16 to 256 energy thresholds are available for energy determination, if each individual pixel 206ᵢ has 4 associated comparators. However, other configurations are of course possible. Preferably, the individual pixels 206ᵢ forming a superpixel are arranged in a rectangular array, which e.g. comprises between 4 x 4 and 8 x 8 individual pixels. Hereby, equal spatial resolutions in the x-direction and y-direction can be achieved.

When individual pixels 206i are interconnected to form a superpixel, the superpixel is the smallest spatial unit for localizing detection events. Therefore, the spatial resolution of the radiation detector 1 is reduced when the superpixels are formed. However, the remaining spatial resolution is usually still sufficient for gamma imaging, which generally requires a lower spatial resolution compared with x-ray imaging. So, a lower spatial resolution is generally sufficient to generate accurate gamma images. Moreover, noise can be reduced, when larger (super-) pixels are used for the detection, which register a higher number of photons compared with smaller pixels

The superpixels are employed only in gamma imaging. In x-ray imaging, the pixels 206ᵢ and their associated comparators 209_{i;1,...,N} are not interconnected. Thus, all pixels 206ᵢ are separated in the x-ray imaging mode of the radiation detector 1, and pixels 206ᵢ are interconnected to form superpixels in the gamma imaging mode. In order to realize both modes of operations, the radiation detector 1 comprises a plurality of switches, which allow for selectively interlinking the anode pixels 206ᵢ of each superpixel and the associated comparators 209_{i;1,...,N}.

In a superpixel, the anode contact elements 206ᵢ included therein are electrically interconnected and the interconnected anode contact elements 206ᵢ are connected to one single amplifier/shaper combination. Therefore, the conversion unit 202 includes switches 210 for selectively interconnecting the anode contact elements 206ᵢ of each superpixel. These switches are opened (i.e. in a non-conducting state) in the x-ray imaging mode and closed (i.e. in a conducting state) in the gamma imaging mode. Moreover, the conversion unit 202 includes switches 211 for connecting the anode contact elements 206ᵢ of each superpixel to the common amplifier/shaper combination of the respective superpixel and disconnecting the anode contact elements 206ᵢ from their associated amplifier 207ᵢ (as far as they are not used in the gamma imaging mode). These switches are closed in the x-ray imaging mode and opened in the gamma imaging mode.

In one implementation, the conversion unit 202 includes a dedicated amplifier/shaper combination for each superpixel, which is only used in the gamma imaging mode. This means that in the gamma imaging mode, all anode contact elements 206ᵢ are disconnected from their associated amplifier 207ᵢ employed in the x-ray imaging mode by means of switches 211. In a further implementation, which is illustrated in Fig. 2, each superpixel uses the amplifier/shaper combination associated with one of the anode contact elements 206ᵢ included therein. In this case, only the further anode contact elements 206ᵢ are disconnected from their associated amplifier 207ᵢ by means of the switches 211. The relevant amplifier/shaper combination may be used in the same configuration in the gamma imaging mode and the x-ray imaging mode. Likewise, the relevant amplifier/shaper combination may be operable in different modes, where one mode may be activated in the gamma imaging mode and the other mode may be activated in the x-ray imaging mode. For instance, the modes may differ in the feedback loop of the amplifier which may be adapted to gamma imaging and to x-ray imaging in the different modes.

Further, the amplifier/shaper combination of each superpixel is connected to all comparators 209_{i;1,...,N} associated with the pixels 206ᵢ included in the superpixel in the gamma imaging mode. In the x-ray imaging mode, the comparators 209_{i;1,...,N} are connected to the shaper 208ᵢ of their associated pixel 206ᵢ. In order to achieve this, the counting unit 203 comprises switches 212 for selectively disconnecting the comparators 209_{i;1,...,N} from the shaper 207ᵢ of their associated pixels (as far as these shapers 207ᵢ are not used in the gamma imaging mode). The switches 212 are closed in the x-ray imaging mode and opened in the gamma imaging mode.

Moreover, the counting unit 203 includes switches 213 for selectively connecting the comparators 209_{i;1,...,N} to the amplifier/shaper combination of the superpixel including the pixels to which the comparators 209_{i;1,...,N} are associated. The switches 213 are opened in the x-ray imaging mode and closed in the gamma imaging mode. If the amplifier/shaper combination associated with one of the individual pixels 206ᵢ of a superpixel is also used in the gamma imaging mode as described above, no switch 213 needs to be provided in the path between this amplifier/shaper combination and the comparators 209 _{i;1,...,N} associated with respective pixel 206ᵢ.

The comparators 209_{i;1,...,N} are coupled to an evaluation unit 214 which is configured to determine the number of detection events in each of the successive time frames. For this purpose, the evaluation unit 214 may evaluate the outputs of the comparators associated with the pixels (in the x-ray imaging mode) or superpixels (in the gamma imaging mode) and register for each detection event only the output pulse of the comparator associated with the highest threshold. Alternatively, the evaluation unit 214 may register the output pulses of all comparators 209_{1,...,N} for each detection event and may determine the number of detection events in each energy bin on the basis of a difference between the registered number of pulses output by the comparator associated with this energy bin and the number of registered pulses output by the comparators associated with lower energy bins.

In such a manner, the evaluation unit 214 determines the number of detection events detected in each pixel 206_{1,...,M} (in the x-ray imaging mode) or in each superpixel (in the gamma imaging mode) per available energy bin in each time frame. Upon expiration of each time frame, a reset of the evaluation unit 214 is carried out (i.e. the number of events detected in each energy bin is reset to zero for each pixel 206_{1,...,M} or superpixel), and in the next time frame, the evaluation unit 214 again determines the number of events detected per energy bin in each pixel 206_{1,...,M} or superpixel in this frame.

In the evaluation unit 214, the number of events per pixel or superpixel and per energy bin are determined and processed in a binary format. In this regard, the frame-wise determination of the numbers of photon events allows for limiting the bit length of the numbers determined and processed in the evaluation unit 214. Therefore, the frame length used in the x-ray imaging mode is selected such that the maximum number of expected detection events detected in each energy bin does not exceed the bit length supported by the evaluation unit 214.

In the gamma imaging mode, the frame length is preferably selected such that the average rate of detection events in each superpixel is below one event per frame in order to facilitate the clustering of detection events, as will be described herein below. In particular, this ensures that plural detection events occurring in one frame - which are clustered in the gamma imaging mode - likely occur essentially simultaneously in response to a single incident gamma photon. In order to achieve such a rate of detection events, the same frame length as in the x-ray imaging mode may be used in the gamma imaging mode. However, as the photon rate in nuclear imaging is typically significantly smaller than the photon rate in x-ray imaging, the aforementioned low event rate may still be achieved when using a longer frame rate in the gamma imaging mode compared with the x-ray imaging mode. Therefore, the frame length in the gamma imaging mode may be selected longer than in the x-ray imaging mode. Hereby, the radiation detector 1 can be operated more efficiently in the gamma imaging mode.

As the skilled person knows, the numbers of detection events per pixel or superpixel and per energy bin determined in the aforementioned manner do not correspond to the number of incident photons due to effects such as charge sharing, K-escape and Compton scattering. Due to these effects, a single photon having a certain energy can produce several detection events with smaller energies in the radiation detector 1.

Charge sharing occurs when the charge cloud generated by a photon in the converter element 204 is collected in several neighboring pixels 206_{1,...,M} or superpixels. This may particularly occur when the photon enters the converter element 204 in the boundary region between adjacent pixels 206_{1,...,M} or superpixels. K-escape occurs when a photon induces K-fluorescence in the material of the converter element 204 before being absorbed in this material. In this case, the photon interacts with the material of the converter element 204 to excite one or more atoms to emit fluorescence photons and looses part of its energy in these processes before being absorbed in the converter element 204. The emitted fluorescence photons are then registered in neighboring parts of the converter element 204, which may belong to other pixels 206_{1,...,M} or superpixels. When a photon undergoes Compton scattering, a part of its energy is absorbed in the converter element 204 at one location during the scattering process. Thereupon, the photon may further travel through the converter element 204 until the remaining photon energy is absorbed in another part of the converter element 204, which may belong to another pixel 206_{1,...,M} or superpixel.

All these effects result in situations in which one photon causes detection events registered in several pixels 206_{1,...,M} or superpixels of the converter element 204, where the energy of the incident photon corresponds to the cumulated energy of these detection events. As a consequence, the radiation detector 1 does not detect the correct number of photons and it does also not correctly determine the energy of all photons.

As explained in the introduction, the resulting inaccuracies in the measurement data provided by the radiation detector 1 in lower energy x-ray imaging only affect a relatively small fraction of detection events and may efficiently be corrected for using model-based correction approaches. However, in gamma imaging, greater inaccuracies occur. This is particularly due to the fact that a larger number of Compton scattering events occur due to the higher photon energy. Such greater inaccuracies even occur when pixels 206_{1,...,M} are interlinked to form superpixels in the gamma imaging mode, as described above. Since these superpixels are larger than the individual pixels, the aforementioned effects are mitigated. However, they may still distort the measurement data acquired in the gamma imaging mode to a greater extent, which is often not acceptable.

In the gamma imaging mode, a clustering of detection events, which are registered in the radiation detector 1, is therefore carried out in order to correct photon numbers and energies which have been incorrectly determined due to the aforementioned effects. The clustering is performed in a clustering unit 215, which may process the measurement data of each time frame in the gamma imaging mode, i.e. the number of detection events registered by the evaluation unit 214 per energy bin in each superpixel. In so doing, the clustering unit 215 identifies detection events, which have been detected in neighboring superpixels of the radiation detector 1. Such events are also referred to herein as cluster events. These cluster events are assumed to have been caused by a single photon which produced detection events in several pixels due to one of the aforementioned effects. In one embodiment, the clustering unit 215 is included in the radiation detector 1, particularly in the ASIC of the radiation detector 1. In this case, the reconstruction unit 5 receives the results of the clustering process, i.e. the information about the detected cluster events, including for each cluster event the associated energy value and an associated virtual location determined as will be explained in further detail herein below. In an alternative embodiment, the clustering unit 215 is provided external of the radiation detector 1. In this embodiment, the clustering unit 215 may be included in the reconstruction unit 5, for example.

In one implementation, a cluster event is detected if a first event has been detected in a first superpixel and if one or more further event(s) has/have been detected in a neighborhood of a first superpixel in the same time frame. These detection events together are then regarded as a cluster event. The neighborhood may include the nearest neighbor superpixels of the first superpixel and may additionally include the next-nearest neighbor superpixels of the first superpixel. Thus, if the superpixels are arranged in perpendicular rows and columns, the neighborhood may correspond to a square of 3 x 3 superpixels or of 5 x 5 superpixels having the first superpixel in its center.

For each detected cluster event, the clustering unit 215 may determine an energy value and a virtual detection location. With respect to the energy value, it is noted that the cumulated energy of multiple detection events caused by a single incident photon due to the aforementioned effects corresponds to the initial energy of this photon, when it enters the radiation detector 1. In view of this, the energy value for a cluster event is determined based on the energies determined for the individual detection events included in the cluster event. In particular, the energy value of the cluster event may correspond to the sum of the energy values determined for the individual detection events included in a cluster event. These energy values are ascertained on the basis of the energy bins associated with the individual detection events included in the cluster event. In one related implementation, each of these energy values corresponds to a predetermined energy value of the energy bin associated with the respective individual detection event, where the predetermined energy values may particularly correspond to the center values of the energy bins. Thus, if a cluster event comprises a detection event to which an energy bin from 90 to 100 keV has been assigned and a further detection event to which an energy bin from 40 to 50 keV has been assigned, an energy value of 95 keV + 45 keV = 140 keV may be assigned to the cluster event, for exmaple.

In order to be able to determine the overall energy of a cluster event in this manner, it is necessary to be able to accurately determine the energy of each detection event included in the cluster event, where each detection can potentially have an arbitrary energy value between a very low energy value and a maximum energy, which corresponds to the energy of the gamma photons emitted by the radioisotope introduced into the object to be imaged. In order to be able to accurately determine the energies of the detection events, a higher number of energy bins is used for the energy determination in the gamma imaging mode than in the x-ray imaging mode. As explained above, this is achieved by interlinking the comparators of the anode pixels 206_{1,...,M} included in each superpixel so that a higher number of different energy thresholds is available. In each superpixel, the thresholds may be selected such that the energy range up to the maximum energy is partitioned into equal intervals forming the energy bins. The thresholds may correspond to lower or upper end values of these intervals.

If Tc-99m is used as radioisotope, the maximum energy of the gamma photons is about 150 keV. Further, if each individual anode pixel 206_{1,...,M} has three associated comparators (in the x-ray imaging mode) and if each superpixel includes 8 x 8 individual anode pixels, 3 x 8 x 8 = 192 thresholds are available. In this case, the energy range from 0 to 150 keV may be portioned into 192 energy intervals or bins, each having a width of 780,125 eV.

The virtual location of the cluster event is determined based on the superpixels in which the detection events included in the cluster event have been detected. In one implementation, the virtual location assigned to a cluster event corresponds to the superpixel where the highest energy is deposited, i.e. the superpixel in which the detection event with the highest energy among the detection events included in the cluster event has been registered. This approach is based on the - often valid - assumption that a gamma photon deposits the largest part of its energy at the location of its first interaction with the material of the conversion unit 204. In alternative implementations, the virtual location may correspond to a spatial average position of the centers of the superpixels, in which the individual photon events included in the cluster have been detected. The spatial average position may correspond to the center point of the centers of the relevant superpixels or to a weighted average position, where the weights may be determined on the basis of the energies deposited in the relevant superpixels.

As a result of the formation of superpixels, the spatial resolution of the radiation detector 1 is reduced in the gamma imaging mode compared with the x-ray imaging mode. This affects the accuracy of the localization of all events detected in the gamma imaging mode, also including the detection of "normal" photon events (i.e. detection events corresponding to a photon that deposits its complete energy in the conversion unit 204 in one single interaction with the semiconductor material of the conversion unit 204). Moreover, the virtual location determined for a cluster event as described above will at least in a certain number of cases not correspond to the superpixel at which the gamma photon producing the detection events are included in the cluster event. This further reduces the accuracy of the localization of photon events in the gamma imaging mode. However, localization of photon events is usually not critical in nuclear imaging applications such as SPECT and the accuracy achievable by means of the radiation in the gamma imaging mode is usually still sufficient for nuclear imaging.

If the superpixels include 8 x 8 anode pixels 206_{1,...,M} with a size of 150 µm, for example, "normal" photon events can still be localized with a spatial accuracy of 1,2 mm. Moreover, cluster events which are detected in neighboring superpixels can still be localized with an accuracy of 3,6 mm or less (depending on how the detection events included in the cluster events are spread over the radiation detector 1). Such an accuracy is usually sufficient for nuclear imaging modalities such as SPECT imaging.

In the process of reconstructing nuclear images, the detected cluster events may be evaluated on the basis of their associated energies and virtual locations in the same manner as "normal" photon events. In one implementation, the image reconstruction may be carried on the basis of unscattered photons, i.e. photons which do not undergo (Compton) scattering before the reach the radiation detector 1. Such photons reach the radiation detector with an energy corresponding to the gamma-ray line emission of the applied radioisotope. Therefore, the reconstruction unit 5 may reconstruct nuclear images only the basis of "normal" photon events and cluster events having an energy in a certain (small) range around the gamma ray emission line of the radioisotope.

Variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage. Any reference signs in the claims should not be construed as limiting the scope

## Claims

1. An imaging system for imaging an object,
the imaging system including an x-ray source (4) and a spectral radiation detector (1) for detecting x-ray and gamma photons and
the system being operable in an x-ray imaging mode, in which the radiation detector (1) is operated to register detection events caused by incident x-ray photons emitted by the x-ray source (4) and having traversed the object, and in a gamma imaging mode, in which the radiation detector (1) is operated to register detection events caused by incident gamma photons emitted by the object,
the radiation detector (1) comprising
a conversion unit (202) including a plurality of detector pixels (206_{1,...,M}) and configured for generating for each detection event a detection signal indicative of an energy of the detection event, and
a counting unit (203) including for each detector pixel (206_{1,...,M}) a plurality of comparators (209_{i;1,...,N}) and configured for associating each detection event to one of a plurality of predetermined energy bins on the basis of the detection signals using the comparators (209_{i;1,...,N}),
wherein, in the x-ray imaging mode, the comparators (209_{i;1,...,N}) of one pixel (206_{1,...,M}), and, in the gamma imaging mode, the comparators (209_{i;1,...,N}) of several pixels (206_{1,...,M}) are available for the association so that the number of energy bins available for the association in the gamma imaging mode is larger than the number of energy bins available for the association in the x-ray imaging mode.

2. The imaging system as defined in claim 1, wherein each comparator (206_{1,...,M}) is configured to compare a value of the detection signal with a threshold value (S_{i;1,...,N}) corresponding to one of the energy bins.

3. The imaging system as defined in claim 2, wherein the radiation detector (1) comprises a switch assembly (210, 211, 212, 213) for interconnecting a set of pixels (206_{1,...,M}) to form a superpixel producing a related detection signal in response to each detection event occurring in the set of pixels (206_{1,...,M}) and for coupling all comparators (209_{i;1,...,N}) associated with the pixels (206_{1,...,M}) included in the set to receive the detection signal in the gamma imaging mode.

4. The imaging system as defined in claim 2 or 3, wherein each pixel (206_{1,...,M}) produces a detection signal in response to a detection event occurring therein and only the comparators (209_{i;1,...,N}) associated with the respective pixel (206_{1,...,M}) receive the detection signal in the x-ray imaging mode.

5. The imaging system as defined in claim 1, further comprising a clustering unit (215) configured to detect a cluster event comprising detection events essentially simultaneously detected at neighboring locations of the radiation detector (1) and to assign an energy value to each detected cluster event on the basis of the energy bins associated with the detection events included in the cluster event in the gamma imaging mode.

6. The imaging system as defined in claim 3 and 5, wherein the clustering unit (215) is configured to detect a cluster event comprising detection events essentially simultaneously detected in neighboring superpixels.

7. The imaging system as defined in claim 5, wherein the energy value assigned to the cluster event corresponds to a sum of predetermined energy values of the energy bins associated with the detection events included in the cluster event, particularly to a sum of center values of the energy bins.

8. The imaging system as defined in claim 5, wherein the clustering unit (215) is configured to assign a virtual detection location to a cluster event on the basis of the locations at which the detection events included in the cluster event are detected.

9. The imaging system as defined in claim 8, wherein the virtual location assigned to a cluster event corresponds to a location at which the detection event with the highest associated energy among the detection events included in the cluster event is detected.

10. The imaging system as defined in claims 3 and 5, wherein the clustering unit (215) is configured to assign a virtual detection location to a cluster event on the basis of the superpixels in which the detection events included in the cluster event are detected.

11. The imaging system as defined in claim 5, wherein the spectral radiation detector (1) comprises an application-specific integrated circuit including the counting unit (203) and the clustering unit (215).

12. The imaging system as defined in claim 1, wherein the system is operable in a hybrid imaging mode in which the x-ray source (4) and the radiation detector (1) rotate around the object to be imaged and register detection events at a plurality of angular positions, at each position the radiation detector (1) being operated in the x-ray imaging mode in a first time interval and in the gamma imaging mode in a second time interval.

13. The imaging system as defined in claim 12, wherein the system is configured to operate the x-ray source (4) only in the first time interval at each angular position.

14. An imaging method for imaging an object using an imaging system including an x-ray source (4) and a spectral radiation detector (1) for detecting x-ray and gamma photons,
the system being operable in an x-ray imaging mode, in which the radiation detector (1) is operated to register detection events caused by incident x-ray photons emitted by the x-ray source (4) and having traversed the object, and in a gamma imaging mode, in which the radiation detector (1) is operated to register detection events caused by incident gamma photons emitted by the object,
the method comprising
a conversion unit (202) of the radiation detector (1), which includes a plurality of detector pixels (206_{1,...,M}), generating for each detection event a detection signal indicative of an energy of the detection event, and
a counting unit (203), which includes for each detector pixel (206_{1,...,M}) a plurality of comparators (209_{i;1,...,N}), associating each detection event to one of a plurality of predetermined energy bins on the basis of the detection signals using the comparators (209_{i;1,...,N}),
wherein in the x-ray imaging mode, the comparators (209_{i;1,...,N}) of one pixels (206_{1,...,M}), and, in the gamma imaging mode, the comparators (209_{i;1,...,N}) of several pixels (206_{1,...,M}) are used for the association so that the number of energy bins available for the association in the gamma imaging mode is larger than the number of energy bins available for the association in the x-ray imaging mode.
